Europäisches Patentamt

European Patent Office

Office européen des brevets

(19)

(11) Veröffentlichungsnummer : **0 384 267 B1**

(12) **EUROPÄISCHE PATENTSCHRIFT**

(45) Veröffentlichungstag der Patentschrift :
26.05.93 Patentblatt 93/21

(51) Int. Cl.$^5$ : **A61M 35/00, A61L 15/00**

(21) Anmeldenummer : **90102771.4**

(22) Anmeldetag : **13.02.90**

(54) **Therapeutisches System zur verzögerten und gesteuerten transdermalen oder transmucosalen Verabreichung von Wirkstoffen.**

(30) Priorität : **18.02.89 DE 3905051**

(43) Veröffentlichungstag der Anmeldung :
**29.08.90 Patentblatt 90/35**

(45) Bekanntmachung des Hinweises auf die
Patenterteilung :
**26.05.93 Patentblatt 93/21**

(84) Benannte Vertragsstaaten :
**AT BE CH DE DK ES FR GB GR IT LI LU NL SE**

(56) Entgegenhaltungen :
**GB-A- 2 174 605**
**US-A- 4 781 924**
**JOURNAL OF CONTROLLED RELEASE, Band 6, Dezember 1987, Seiten 107-111; NL; C.D.E-BERT et al.: "Development of a novel trans-dermal system design"**

(73) Patentinhaber : **LTS Lohmann**
**Therapie-Systeme GmbH & Co. KG**
**Irlicherstrasse 55**
**W-5450 Neuwied 12 (DE)**

(72) Erfinder : **Müller, Walter, Dr. Dipl.-Chem.**
**Engerser Strasse 56**
**W-5450 Neuwied 1 (DE)**
Erfinder : **Kindel, Heinrich**
**Westerwaldstrasse 7**
**W-5455 Engsdorf (DE)**

(74) Vertreter : **Flaccus, Rolf-Dieter, Dr.**
**Patentanwalt Sperlingsweg 32**
**W-5047 Wesseling (DE)**

**Beschreibung**

Die Erfindung betrifft eine Vorrichtung zur verzögerten und gesteuerten transdermalen oder transmucosalen Verabreichung von Wirkstoffen in Form eines therapeutischen Systems nach der Angaben des Anspruchs 1.

Unter therapeutischen Systemen sind arzneistoffhaltige Vorrichtungen bzw. Darreichungsformen zu verstehen, die einen Arzneistoff oder mehrere in vorausbestimmter Rate kontinuierlich über einen festgelegten Zeitraum an einen festgelegten Anwendungsort abgeben (siehe Heilmann, "Therapeutische Systeme", Enke Verlag, Stuttgart, 1984, Seite 24). Ein transdermales therapeutisches System gibt den Wirkstoff über die Haut ab und wird demgemäß auf der Haut, also topisch angewandt.

Ziel bei der Anwendung von Wirkstoffen sollte immer sein, den Wirkstoff in einer Menge zu verabreichen, die einerseits möglichst niedrig ist, andererseits aber mit größtmöglicher Sicherheit den gewünschten therapeutischen Effekt erwarten läßt. Zu diesem Zweck wurde eine ganze Reihe von sogenannten therapeutischen Systemen entwickelt, die den Wirkstoff auf eine durch die Systemparameter vorgegebene Weise gesteuert abgeben. Für eine ganze Reihe von systemisch wirkenden Wirkstoffen besteht nicht die Notwendigkeit oder der Wunsch, über den gesamten Zeitraum eines Tages gleichmäßig hohe Plasmaspiegel zu haben.

So ist es z.B. für einige Wirkstoffe von Vorteil, wenn die Wirkstoffspiegel während der Nachtruhe möglichst niedrig liegen und erst gegen Ende der Schlafperiode auf das therapeutisch notwendige Niveau angehoben werden.

Dabei ist besonders gedacht an Nitroverbindungen zur Angina pectoris Prophylaxe, da erstens Angina pectoris Anfälle in der Nacht selten und in den frühen Morgenstunden vergleichsweise häufig auftreten, und zweitens die bei diesen Nitroverbindungen mögliche Toleranzentwicklung schon durch eine mehrstündige Unterbrechung der Medikation vermieden werden kann. Aber auch für Nicotin, Appetitzügler, blutdruckbeeinflußende Mittel (β-Blocker) oder Antiasthmatika (β-Sympathomimetika) wäre eine dermaßen auf den Bedarf angepaßte Dosierung wünschenswert.

Eine Arzneiform, die dieses leistet, muß die Wirkstoffabgabe an den Organismus nach der abendlichen Verabreichung um etwa 4-10 Stunden verzögern, so daß sich dann ohne weiteres Zutun des Patienten gegen Ende der Schlafphase therapeutisch notwendige Plasmaspiegel einstellen würden.

Besonders geeignet für diese doch recht lange Verzögerungszeit ist die transdermale oder transmucosale Verabreichung von Wirkstoffen. In der US-PS 4,655,766 wurde ein auf osmotischen Prinzipien beruhendes transdermales System, in der EP-A 0249475 ein auf Diffusionsprozessen basierendes System und in der EP-A 0249343 ein System beschrieben, welches erst durch die Zufuhr von Flüssigkeiten, wie z.B. der Hautfeuchtigkeit, aktiviert wird.

Systeme gemäß EP-A 0249475 bestehen prinzipiell aus zwei getrennten Teilen, dem eigentlichen Wirkstoffreservoir und einem flächenförmigen wirkstofffreien Teil. Vor der Applikation wird die wirkstofffreie Schicht flächendeckend auf die Freigabeseite des Reservoirs aufgebracht, und dann das System mit der anderen Seite dieser wirkstofffreien Schicht auf die Haut geklebt. Zwischen den beiden Teilen des Systems besteht also ein Konzentrationsgradient bezüglich des Wirkstoffs. Dies hat nach den Fick'schen Diffusionsgesetzen zur Folge, daß der Wirkstoff in diese wirkstofffreie Schicht hineindiffundiert und nach einer durch die Systemparameter vorgegebenen Zeit auch die Haut erreicht. Die Verzögerungszeit entspricht also der Zeitspanne, die verstreicht vom Zeitpunkt der Vereinigung der vor Applikation getrennten Systemteile bis zu dem Zeitpunkt, ab dem der Wirkstoff mit einer für den therapeutischen Zweck ausreichenden Menge an die Haut abgegeben wird.

In der EP-A 0249475 werden für die Berechnung dieser Zeitspanne zwei Formeln angegeben. Formel (1) ist gültig für ein Reservoir ohne Membransteuerung

$$T = L^2/6D \quad (1)$$

und Formel (2) für ein Reservoir mit Membransteuerung, d.h. für ein Reservoir mit konstantem Flux

$$T = (L \times C) / (6 \times J) \quad (2).$$

| | |
|---|---|
| $T$ (h) | Verzögerungszeit |
| $L$ (cm) | Dicke der Verzögerungsschicht |
| $D$ (cm^2/ h) | Diffusionskoeffizient des Wirkstoffs in der Verzögerungsschicht |
| $C$ (μg/ cm^3) | Sättigungskonzentration des Wirkstoffs in der Verzögerungsschicht |
| $J$ (μg/ h) | Wirkstofflux aus dem Reservoir |

Laut Formel (1) stehen als Parameter zur Beeinflussung der Verzögerungszeit die Dicke der Verzögerungsschicht und der Diffusionskoeffizient des Wirkstoffs zur Verfügung. Eine Verlängerung der Verzögerungszeit wird erreicht durch eine Erhöhung der Schichtdicke und eine Erniedrigung des Diffusionskoeffizienten.

Da die maximale Durchlaßkapazität sich nach folgender Formel

2

$$J(max) = (D \times C) / L \quad (3)$$

berechnet, ist damit gezeigt, daß eine lange Verzögerungszeit automatisch den Wirkstoffflux begrenzt, wenn die Abgabekapazität des Reservoirs über der Durchlaßkapazität der Verzögerungsschicht liegt.

Dies ist ein schwerer den Einsatz solcher Systeme limitierender Nachteil.

Dieser prinzipielle Nachteil existiert nicht bei Systemen gemäß EP-A 0249343.

Hier wird die Wirkstoffabgabe aus dem Reservoir durch eine Membran gesteuert, die eine gewisse Zeit nach Applikation des Systems ihre Durchlaßkapazität durch die Aufnahme einer Aktivatorflüssigkeit stark erhöht und so praktisch von wirkstoffundurchlässig auf wirkstoffdurchlässig geschaltet wird. Die Aktivatorflüssigkeit ist entweder die Hautfeuchtigkeit selbst, eine Flüssigkeit, in die das System vor Gebrauch eingetaucht wird, oder die das System in einem getrennten Reservoir enthält und erst nach einer zusätzlichen Manipulation freigibt.

Nachteilig bei diesem System ist jedoch, daß es schwierig ist, eine solche Membran zeitlich vorprogrammiert bezüglich ihrer Permeabilität für den Wirkstoff zu ändern, insbesondere dann, wenn die Hautfeuchtigkeit als Aktivator eingesetzt wird. In diesem Fall spielen große individuelle Unterschiede und schlecht zu kontrollierende Parameter, wie z.B. Raumtemperatur oder Bekleidung eine große Rolle und beeinflussen unkontrollierbar die Länge der Verzögerungszeit und den Wirkstoffflux aus dem System.

Aufgabe der Erfindung war es nun, Systeme zur verzögerten transdermalen oder transmucosalen Verabreichung von Wirkstoffen zu entwickeln, die die hohe prinzipielle Funktionssicherheit der Systeme gemäß der EP-A 0249475 mit dem Vorteil des durch das Verzögerungselement nur wenig beeinflußten Wirkstofffluxes von Systemen gemäß EP-A 0249343 miteinander vereinen.

Die Lösung dieses Problems bestand überraschenderweise darin, in die wirkstofffreie Verzögerungsschicht einen Hilfsstoff einzuarbeiten, der bis zu seiner Erschöpfung oder bis zum Eintreten eines Gleichgewichtszustandes den Wirkstoff in eine nicht bioverfügbare Form überführt.

Diese wirkstofffreie Verzögerungsschicht darf selbstverständlich erst unmittelbar vor oder nach der Applikation mit dem Wirkstoffreservoir in Kontakt gebracht werden und befindet sich nach der Applikation zwischen Reservoir und der Haut bzw. der Schleimhaut.

Erreichen kann man dies z.B. dadurch, daß man zuerst die Verzögerungsschicht auf den Applikationsort klebt und erst danach darauf das Reservoir oder, wie in der EP-A 0249475 beschrieben, durch die Herstellung des Gesamtsystems unmittelbar vor der Applikation.

Gleichung 1 und 2 zeigen, daß die Verzögerungszeit proportional der dem Quadrat der Schichtdicke bzw. direkt proportional der Schichtdicke ist. Andererseits geht aus der Gleichung 3 eindeutig hervor, daß die maximale Durchlaßkapazität einer Schicht umgekehrt proportional der Schichtdicke ist.

Dies bedeutet, daß die Durchlaßkapazität umso kleiner wird, je länger die Verzögerungszeit sein soll.

Dadurch, daß ein Teil des in die Verzögerungsschicht eindringenden Wirkstoffs in eine nicht bioverfügbare Form überführt wird - und dies ist natürlich der zuerst eindiffundierende Wirkstoffteil - erhält man eine lange Verzögerungszeit, ohne relativ dicke Verzögerungsschichten verbunden mit einer erniedrigten Durchlaßkapazität in Kauf nehmen zu müssen.

Systeme im Sinne dieser Erfindung bieten prizipeill zwei Möglichkeiten, den Wirkstoff in eine nicht bioverfügbare Form zu überführen:

a. der Wirkstoff wird immobilisiert

b. der Wirkstoff wird in ein Salz überführt.

Immobilisiert wird der Wirkstoff immer dann, wenn er an ein selbst nicht zur Diffusion befähigtes Polymer gebunden wird. Dies ist der Fall, wenn der Wirkstoff eine Säure oder eine Base ist und in der Verzögerungsschicht an eine polymere Polybase bzw. Polysäure gebunden wird. Der Wirkstoff wird dabei über eine einfache Säure-Base Reaktion in ein polymeres Salz überführt und kann in dieser Form das System nicht verlassen.

$$B + \text{polym. Polysäure} \longrightarrow BH \overset{+-}{\quad} O_2C\text{--} \overset{\text{\textbackslash}}{\text{\textbar}}\text{\textbackslash Polymer}$$

$$B = \text{Wirkstoffbase}$$

$$HS + \text{polym. Polybase} \longrightarrow S \overset{-+}{\quad} HB\text{--} \overset{\text{\textbackslash}}{\text{\textbar}}\text{\textbackslash Polymer}$$

$$S = \text{Wirkstoffsäure}$$

Aber auch wenn der Wirkstoff selbst als ein zur Penetration in die Haut befähigtes Salz vorliegt, kann er immobilisiert werden, wenn das wirksame Ion über einen Ionentauscher gegen ein unwirksames Ion ausgetauscht wird.

Unspezifisch wirken solche Hilfsstoffe, die über physikalische Wechselwirkungen in der Lage sind, Substanzen an ihrer Oberfläche zu absorbieren. Zu diesen Substanzen gehören z.B. Kieselgel oder Aluminiumoxid, die aufgrund dieser Fähigkeiten verbreitet Anwendung in der Chromatographie finden.

Speziell bei transdermaler Applikation von Wirkstoffen ist die lipophile Barriere des Stratum Corneum zu überwinden. Dies ist naturgemäß besonders schwierig für Substanzen, die polarer oder ionischer Natur sind. Da die meisten Wirkstoffe entweder selbst Basen oder Säuren sind, ist es ohne weiteres möglich, sie durch Salzbildung in der Verzögerungsschicht so hydrophil zu machen, daß sie dann als nunmehr ionische Verbindungen zwar zur Diffusion im System befähigt sind, aber nicht mehr das Stratum Corneum passieren können und damit also nicht mehr bioverfügbar sind.

Charakteristisch für alle diese Reaktionen ist, daß die sich mit dem Wirkstoff in der Verzögerungsschicht umsetzenden Hilfsstoffe dies nur tun, bis sie entweder erschöpft sind oder bis sich ein Gleichgewicht eingestellt hat. Erst ab diesem Zeitpunkt gelangt Wirkstoff auch in bioverfügbarer Form an den Applikationsort, d.h. an die Haut oder Schleimhaut.

Die Verzögerungszeit wird also wesentlich bestimmt durch die Lieferkapazität des Wirkstoffreservoirs einerseits und die Menge des mit dem Wirkstoff reagierenden Hilfsstoffs in der Verzögerungsschicht andererseits. Durch die Erhöhung der Hilfsstoffkonzentration ist es ohne weiteres möglich, die Verzögerungszeit bei gleichbleibender Schichtdicke zu verlängern. Selbstverständlich kann sowohl das Reservoir als auch die Verzögerungsschicht beliebig kompliziert, z.B. mehrschichtig aufgebaut sein. Insbesondere kann es von Vorteil sein, den Wirkstofffluß steuerende Membranen zwischen Wirkstoffreservoir und Verzögerungsschicht und/oder zwischen Verzögerungsschicht und Applikationsort einzufügen.

Ebenfalls kann es sinnvoll sein, nicht- oder nur schwachklebende Schichten zu vewenden, die dann, wenn nötig mit zusätzlichen selbstklebenden Filmen versehen werden müssen.

Als Materialien für die Reservoire bzw. für die Rückschicht und die wieder ablösbare Schutzschicht können alle Materialien verwendet werden, die für die Herstellung von transdermalen und transmucosalen Systemen üblich und dem Fachmann hinreichend bekannt sind.

Als Materialien für die Rückschicht geeignet sind z.B. Folien aus Polyester, PVC, Polyamid, Polyethylen oder Polypropylen. Gebräuchlich sind auch Verbundfolien aus diesen Materialien, wobei oftmals eine zusätzliche Aluminiumschicht für die Undurchlässigkeit für Wirkstoffe sorgt.

Als Materialien für die wieder ablösbare Schutzschicht sind prinzipiell die gleichen Materialien wie für die Rückschicht verwendbar, die jedoch zusätzlich adhäsiv ausgerüstet sein müssen.

Als Grundmaterialien für die Reservoire seien beispielhaft genannt: Polyisobutylene, Styrol-Isopren-Styrol Blockpolymerisate, Polysiloxane, Polymethacrylate, Polyurethane, Polyester, Polyamide und Copolymerisate des Ethylens mit z.B. Vinylacetat oder Acrylsäurederivaten.

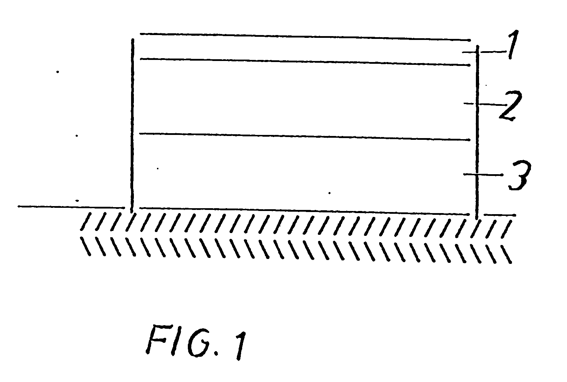

Die Erfindung wird durch die Figur 1 und das nachfolgende Beispiel näher erläutert, wobei in der Figur 1 (1) die undurchlässige Rückschicht, (2) die wirkstoffhaltige Reservoirschicht und (3) die wirkstofffreie Verzögerungsschicht darstellt.

Beispiel

## Herstellung des Wirkstoffreservoirs

300 g      einer 20%-igen Lösung von Oppanol B100 (Polyisobutylen, mittleres MG 1 270 000)

108 g      einer 50%-igen Lösung von Abitol (hydrierter Abietylalkohol) und

108 g      einer 50%-igen Lösung von Piccotac C-BHT (Kohlenwasserstoffharz) in Benzin und

12 g      Miglyol 812 werden zusammengegeben, homogenisiert und anschließend unter Rühren 169 g Lactose (EP D80, Fa. Meggle) mit einer Nitroglycerinbeladung von 10% zugefügt.

Mit dieser Masse wird auf silikonisiertes Kraftpapier ein 350 μ dicker Film gestrichen und das Lösemittel durch 20 minütiges Trocknen bei 50°C entfernt. Der trockene Film hat ein Flächengewicht von 220 g/m$^2$.

Dieser Reservoirfilm klebt aufgrund seines hohen Lactosegehalts verhältnismäßig schlecht, so daß vor dem Aufbringen der aluminisierten Trägerfolie aus Polyester ein klebender Film mit einem Flächengewicht von 20 g/m$^2$ zukaschiert werden muß. Dieser klebende Film enthält keine Lactose, entspricht aber in seiner Zusammensetzung ansonsten dem Reservoir.

## Herstellung der Verzögerungsschicht

300 g      einer 20%-igen Lösung von Oppanol B100,

108 g      einer 50%-igen Lösung von Abitol und

108 g      einer 50%-igen Lösung von Piccotac C-BHT in Benzin und

159 g      Benzin werden zusammengegeben, homogenisiert und anschließend unter Rühren

44 g      Kieselgel 60 H für die Dünnschichtchromatographie (Fa. Merck, Darmstadt) zugegeben. Mit dieser Masse wird ein 250 μ dicker Film auf silikonisiertes Kraftpapier gestrichen und 20 Minuten bei 50°C getrocknet. Der trockene Film hat ein Flächengewicht von 54 g/m$^2$. Auch dieser Film klebt aufgrund seines hohen Feststoffgehalts relativ schlecht, so daß ein besser klebender Film gleicher Zusammensetzung, allerdings ohne Kieselgel, mit einem Flächengewicht von 20 g/m$^2$ zukaschiert werden muß. Das Laminat wird mit einer 100 μ dicken aluminisierten und silikonisierten Polyesterfolie - der wiederentfernbaren Schutzschicht - abgedeckt.

## Durchführung der in-vitro Freisetzung

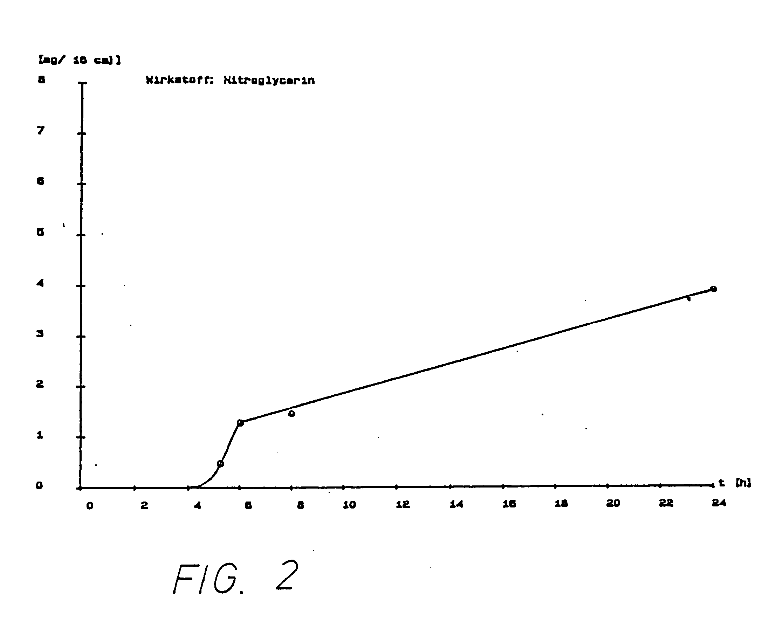

Unmittelbar vor Versuchsbeginn wurden Reservoirschicht und Verzögerungsschicht zusammenkaschiert und in 16 cm$^2$ große Einzelstücke geschnitten. Die in-vitro Freisetzung wurde bei 32°C in einer Rotating Bottle Apparatur unter Verwendung von 100 ml physiologischer Kochsalzlösung als Freisetzungsmedium durchgeführt. Die Proben wurden mittels einer HPLC-Methode vermessen. Der Freisetzungsverlauf ist in Fig. 2 dargestellt.

Es ist deutlich erkennbar, daß erst nach 5 Stunden ein merklicher Wirkstofffluß einsetzt.

## Patentansprüche

**Patentansprüche für folgende Vertragsstaaten : AT, BE, CH, LI, DE, DK, FR, GB, IT, LU, NL, SE**

1. Therapeutisches System zur verzögerten und gesteuerten transdermalen oder transmucosalen Verabreichung von Wirkstoffen, wobei das system aus einer undurchlässigen Rückschicht, einem wirkstoffhaltigen Reservoir, einer im wesentlichen wirkstofffreien aber wirkstoffdurchlässigen Schicht, und einer wieder ablösbaren Schutzschicht besteht, und die wirkstofffreie Schicht erst unmittelbar vor oder nach der Applikation in Kontakt mit dem Wirkstoffreservoir gebracht wird und dann zwischen Reservoir und der Applikationsfläche liegt, <u>dadurch gekennzeichnet</u>, daß die wirkstofffreie Schicht einen Hilfsstoff enthält, der wenigstens einen Teil des nach Kontakt mit dem Wirkstoffreservoir in sie eindiffundierenden Wirkstoffs in eine nicht bioverfügbare Form überführt.

2. Therapeutisches System gemäß Anspruch 1, <u>dadurch gekennzeichnet</u>, daß der Wirkstoff eine Base ist und die wirkstofffreie Schicht eine Säure enthält.

3. Therapeutisches System gemäß Anspruch 1 und 2, <u>dadurch gekennzeichnet</u>, daß die Säure eine polymere Polysäure ist.

4. Therapeutisches System gemäß Anspruch 1 und 2, dadurch gekennzeichnet, daß die wirkstofffreie Schicht einen sauer eingestellten Puffer enthält.

5. Therapeutisches System gemäß Anspruch 1, dadurch gekennzeichnet, daß der Wirkstoff eine Säure ist und die wirkstofffreie Schicht eine Base enthält.

6. Therapeutisches System gemäß Anspruch 1 und 5, dadurch gekennzeichnet, daß die Base eine polymere Polybase ist.

7. Therapeutisches System gemäß Anspruch 1 und 5, dadurch gekennzeichnet, daß die wirkstofffreie Schicht einen basisch eingestellten Puffer enthält.

8. Therapeutisches System gemäß Anspruch 1, dadurch gekennzeichnet, daß der Wirkstoff ein Salz ist und die wirkstofffreie Schicht einen Ionentauscher enthält.

9. Therapeutisches System gemäß Anspruch 1, dadurch gekennzeichnet, daß die wirkstofffreie Schicht einen Hilfsstoff enthält, der aufgrund physikalischer Wechselwirkungen einen Teil des Wirkstoffs zu absorbieren vermag.

10. Therapeutisches System gemäß Anspruch 1 und 9, dadurch gekennzeichnet, daß dieser Hilfsstoff entweder ein Kieselgel, ein Kieselgelderivat, Aluminiumoxid, Aktivkohle, Titandioxid, Zinkoxid, Magnesiumoxid, Lactose oder ein Cellulosederivat ist.

11. Therapeutisches System gemäß Anspruch 1 und 9, dadurch gekennzeichnet, daß der Wirkstoff eine organische Nitroverbindung, bevorzugt Nitroglycerin ist.

12. Therapeutisches System gemäß Anspruch 1 und 9, dadurch gekennzeichnet, daß der Wirkstoff Nicotin ist.

13. Therapeutisches System gemäß Anspruch 1 und 9, dadurch gekennzeichnet, daß der Wirkstoff ein Appetitzügler ist.

14. Therapeutisches System gemäß Anspruch 13, dadurch gekennzeichnet, daß der Appetitzügler Nor-pseudoephedrin, Amfepramon, Mefenorex, Propylhexedrin, Fenfluramin oder Mazindol ist.

15. Therapeutisches System gemäß Anspruch 1 und 9, dadurch gekennzeichnet, daß der Wirkstoff ein Betablocker ist.

16. Therapeutisches System gemäß Anspruch 15, dadurch gekennzeichnet, daß der Betablocker Alprenolol, Oxprenolol, Penbutolol, Bupranolol, Metoprolol, Betaxolol, Atenolol, Acebutolol, Metipranolol, Propranolol, Nadolol, Pindolol, Mepindolol, Carteolol, Carazolol, Timolol oder Sotalol ist.

17. Therapeutisches System gemäß Anspruch 1 und 9, dadurch gekennzeichnet, daß der Wirkstoff zur Gruppe der $\alpha$-Sympathomimetika gehört.

18. Therapeutisches System gemäß Anspruch 17, dadurch gekennzeichnet, daß der Wirkstoff Norfenetrin, Octopamin, Oxedrin, Metaraminol, Midodrin oder Oxilofrin ist.

19. Therapeutisches System gemäß Anspruch 1 und 9, dadurch gekennzeichnet, daß der Wirkstoff zur Gruppe der $\beta$-Sympathomimetika gehört.

20. Therapeutisches System gemäß Anspruch 19, dadurch gekennzeichnet, daß der Wirkstoff Salbutamol, Terbutalin, Fenoterol, Clenbuterol, Reproterol, Hexoprenalin, Bamethan oder Isoxsuprin ist.

**Patentansprüche für folgende Vertragsstaaten : SP, GR**

1. Verfahren zur Herstellung eines therapeutischen Systems zur verzögerten und gesteuerten transdermalen oder transmucosalen Verabreichung von Wirkstoffen aus einer undurchlässigen Rückschicht, wobei ein wirkstoffhaltigen Reservoir, eine im wesentlichen wirkstoffreien aber wirkstoffdurchlässigen Schicht,

und eine wieder ablösbare Schutzschicht vorgesehen sind, und wobei die wirkstofffreie Schicht erst unmittelbar vor oder nach der Applikation in Kontakt mit dem Wirkstoffreservoir gebracht wird und dann zwischen Reservoir und der Applikationsfläche liegt, dadurch gekennzeichnet, daß der wirkstrofffreien Schicht ein Hilfsstoff zugesetzt wird, der wenigstens einen Teil des nach Kontakt mit dem Wirkstoffreservoir in sie eindiffundierenden Wirkstoffs in eine nicht bioverfügbare Form überführt.

2. Verfahren nach Anspruch 1, dadurch gekennzeichnet, daß als Wirkstoff eine Base verwendet und der wirkstofffreien Schicht eine Säure zugesetzt wird.

3. Verfahren nach Anspruch 1 und 2, dadurch gekennzeichnet, daß als Säure eine polymere Polysäure eingesetzt wird.

4. Verfahren nach Anspruch 1 und 2, dadurch gekennzeichnet, daß in der wirkstofffreien Schicht ein sauer eingestellter Puffer eingesetzt wird.

5. Verfahren nach Anspruch 1, dadurch gekennzeichnet, daß als Wirkstoff eine Säure verwendet und der wirkstofffreien Schicht eine Base zugesetzt wird.

6. Verfahren nach Anspruch 1 und 5, dadurch gekennzeichnet, daß als Base eine polymere Polybase eingesetzt wird.

7. Verfahren nach Anspruch 1 und 5, dadurch gekennzeichnet, daß in der wirkstofffreien Schicht ein basisch eingestellter Puffer eingesetzt wird.

8. Verfahren nach Anspruch 1, dadurch gekennzeichnet, daß als Wirkstoff ein Salz verwendet und der wirkstoffreien Schicht ein Ionentauscher zugesetzt wird.

9. Verfahren nach Anspruch 1, dadurch gekennzeichnet, daß der wirkstofffreien Schicht ein Hilfsstoff zugesetzt wird, der aufgrund physikalischer Wechselwirkungen einen Teil des Wirkstoffs zu absorbieren vermag.

10. Verfahren nach Anspruch 1 und 9, dadurch gekennzeichnet, daß als Hilfsstoff entweder ein Kieselgel, ein Kieselgelderivat, Aluminiumoxid, Aktivkohle, Titandioxid, Zinkoxid, Magnesiumoxid, Lactose oder ein Cellulosederivat zugesetzt wird.

11. Verfahren nach Anspruch 1 und 9, dadurch gekennzeichnet, daß als Wirkstoff eine organische Nitroverbindung, bevorzugt Nitroglycerin verwendet wird.

12. Verfahren nach Anspruch 1 und 9, dadurch gekennzeichnet, daß als Wirkstoff Nicotin verwendet wird.

13. Verfahren nach Anspruch 1 und 9, dadurch gekennzeichnet, daß als Wirkstoff ein Appetitzügler verwendet wird.

14. Verfahren nach Anspruch 13, dadurch gekennzeichnet, daß als Appetitzügler Nor-pseudoephedrin, Amfepramon, Mefenorex, Propylhexedrin, Fenfluramin oder Mazindol verwendet wird.

15. Verfahren nach Anspruch 1 und 9, dadurch gekennzeichnet, daß als Wirkstoff ein Betablocker verwendet wird.

16. Verfahren nach Anspruch 15, dadurch gekennzeichnet, daß als Betablocker Alprenolol, Oxprenolol, Penbutolol, Bupranolol, Metoprolol, Betaxolol, Atenolol, Acebutolol, Metipranolol, Propranolol, Nadolol, Pindolol, Mepindolol, Carteolol, Carazolol, Timolol oder Sotalol verwendet wird.

17. Verfahren nach Anspruch 1 und 9, dadurch gekennzeichnet, daß ein Wirkstoff aus der Gruppe der -Sympathomimetika verwendet wird.

18. Verfahren nach Anspruch 17, dadurch gekennzeichnet, daß als Wirkstoff Norfenetrin, Octopamin, Oxedrin, Metaraminol, Midodrin oder Oxilofrin verwendet wird.

19. Verfahren nach Anspruch 1 und 9, dadurch gekennzeichnet, daß ein Wirkstoff aus der Gruppe der $\beta$-Sympathomimetika verwendet wird.

**20.** Verfahren nach Anspruch 19, <u>dadurch gekennzeichnet</u>, daß als Wirkstoff Salbutamol, Terbutalin, Fenoterol, Clenbuterol, Reproterol, Hexoprenalin, Bamethan oder Isoxsuprin verwendet wird.

**Claims**

**Claims for the following Contracting States : AT, BE, CH, LI, DE, DK, FR, GB, IT, LU, NL, SE**

**1.** Therapeutic system for the retarded and controlled transdermal or transmucous administration of active substances, whereby said system comprises an impermeable backing layer, an active substance containing reservoir, a substantially drug-free but drug-germeable layer and a removable protective layer, and whereby the drug-free layer is brought into contact with the active substance reservoir only immediately prior or after application and then lies between reservoir and application surface, characterized in that the drug-free layer contains an auxiliary agent which converts at least a portion of the active substance, diffusing into said layer after contacting the active substance reservoir, into a non-bioavailable form.

**2.** The therapeutic system according to claim 1, characterized in that the active substance is a base and the drug-free layer comprises an acid.

**3.** The therapeutic system according to claims 1 or 2, characterized in that the acid is a polymeric acid.

**4.** The therapeutic system according to claims 1 or 2, characterized in that the drug-free layer comprises an acidified buffer.

**5.** The therapeutic system according to claim 1, characterized in that the active substance is an acid and the drug-free layer comprises a base.

**6.** The therapeutic system according to claims 1 and 5, characterized in that the base is a polymeric base.

**7.** The therageutic system according to claims 1 and 5, characterized in that the drug-free layer comprises a basified buffer.

**8.** The therapeutic system according to claim 1, characterized in that the active substance is a salt and the drug-free layer comprises an ion exchanger.

**9.** The therapeutic system according to claim 1, characterized in that the drug-free layer comprises an auxiliary agent which can absorb a portion of the active substance due to physical interaction.

**10.** The therapeutic system according to claims 1 and 9, characterized in that said auxiliary either is a silica gel, a silica gel derivative, an aluminium oxide, active carbon, titanium dioxide, zinc oxide, magnesium oxide, lactose, or a cellulose derivative.

**11.** The therapeutic system according to claims 1 and 9, characterized in that the active substance is an organic nitro compound, preferably nitroglycerine.

**12.** The therapeutic system according to claims 1 and 9, characterized in that the active substance is nicotine.

**13.** The therapeutic system according to claims 1 and 9, characterized in that the active substance is an appetite-suppressing agent.

**14.** The therapeutic system according to claim 13, characterized in that the appetite-suppressing agent is nor-pseudoephedrine, amfepramone, mefenorex, propylhexedrine, fenfluramine, or mazindol.

**15.** The therageutic system according to claims 1 and 9, characterized in that the active substance is a betablocker.

**16.** The therapeutic system according to claim 15, characterized in that the beta-blocker is alprenolol, oxprenolol, genbutolol, bupranolol, metoprolol, betaxolol, atenolol, acebutolol, metipranolol, propranolol, nadolol, pindolol, mepindolol, carteolol, carazolol, timolol, or sotalol.

**17.** The therapeutic system according to claims 1 and 9, characterized in that the active substance belongs to the group of α-sympathomimetika.

**18.** The therapeutic system according to claim 17, characterized in that the active substance is norfenetrine, octapamine, oxedrine, metaraminol, midodrine, or oxilofrine.

**19.** The therapeutic system according to claims 1 and 9, characterized in that the active substance belongs to the group of β-sympathomimetika.

**20.** The therapeutic system according to claim 19, characterized in that the active substance is salbutamol, terbutaline, fenoterol, clenbuterol, reproterol, hexoprenaline, bamethan, or isoxsuprine.

**Claims for the following Contracting States : ES, GR**

**1.** Process for the production of a therapeutic system for the retarded and controlled transdermal or transmucous administration of active substances, comprising an impermeable backing layer, whereby an active substance-containing reservoir, a substantially drug-free but drug-permeable layer and a removable protective layer, are provided, and whereby the drug-free layer is brought into contact with the active substance reservoir only immediately prior or after application and then lies between reservoir and application surface, characterized in that an auxiliary agent is added to the drug-free layer which converts at least a portion of the active substance, diffusing into said layer after contacting the active substance reservoir, into a non-bioavailable form.

**2.** The process according to claim 1, characterized in that as active substance a base is employed and an acid is added to the drug-free layer.

**3.** The process according to claims 1 or 2, characterized in that as an acid a polymeric acid is employed.

**4.** The process according to claims 1 or 2, characterized in that in the drug-free layer an acidified buffer is employed.

**5.** The process according to claim 1, characterized in that as active substance an acid is employed and a base is added to the drug-free layer.

**6.** The process according to claims 1 and 5, characterized in that as a base a polymeric base is employed.

**7.** The process according to claims 1 and 5, characterized in that in the drug-free layer a basified buffer is employed.

**8.** The process according to claim 1, characterized in that as active substance a salt is employed and an ion exchanger is added to the drug-free layer.

**9.** The process according to claim 1, characterized in that an auxiliary agent which can absorb a portion of the active substance due to physical interaction is added to the drug-free layer.

**10.** The process according to claims 1 and 9, characterized in that as an auxiliary either a silica gel, a silica gel derivative, an aluminium oxide, active carbon, titanium dioxide, zinc oxide, magnesium oxide, lactose, or a cellulose derivative is added.

**11.** The process according to claims 1 and 9, characterized in that as active substance an organic nitro compound, preferably nitroglycerine is employed.

**12.** The process according to claims 1 and 9, characterized in that as active substance nicotine is employed.

**13.** The process according to claims 1 and 9, characterized in that as active substance an appetite-suppressing agent is employed.

**14.** The process according to claim 13, characterized in that as appetite-suppressing agent norpseudoephedrine, amfepramone, mefenorex, propylhexedrine, fenfluramine, or mazindol is employed.

**15.** The process according to claims 1 and 9, characterized in that as active substance a betablocker is employed.

**16.** The process according to claim 15, characterized in that as beta-blocker alprenolol, oxprenolol, penbutolol, bupranolol, metogrolol, betaxolol, atenolol, acebutolol, metipranolol, progranolol, nadolol, pindolol, mepindolol, carteolol, carazolol, timolol, or sotalol is employed.

**17.** The process according to claims 1 and 9, characterized in that an active substance belonging to the group of α-sympathomimetika is employed.

**18.** The process according to claim 17, characterized in that as active substance norfenetrine, octapamine, oxedrine, metaraminol, midodrine, or oxilofrine is employed.

**19.** The process according to claims 1 and 9, characterized in that an active substance belonging to the group of β-sympathomimetika is employed.

**20.** The process according to claim 19, characterized in that as active substance salbutamol, terbutaline, fenoterol, clenbuterol, reproterol, hexoprenaline, bamethan, or isoxsuprine is employed.


**Revendications**


**Revendications pour les Etats contractants suivants : AT, BE, CH, LI, DE, DF, FR, GB, IT, LU, NL, SE**

**1.** Système thérapeutique pour l'administration transdermique, ou transmucosale, retardée et régulée, de substances actives, où le système se compose d'une couche dorsale imperméable, d'un réservoir contenant la substance active, d'une couche sensiblement dépourvue de substance active mais perméable à la substance active et d'une couche protectrice amovible et la couche dépourvue de substance active n'est mise en contact avec le réservoir à substance active qu'immédiatement avant ou après l'application et se situe alors entre le réservoir et la surface d'application, caractérisé en ce que la couche dépourvue de substance active contient un adjuvant qui convertit au moins une partie de la substance active qui y diffuse après contact avec le réservoir à substance active en une forme non biodisponible.

**2.** Système thérapeutique suivant la revendication 1, caractérisé en ce que la substance active est une base et la couche dépourvue de substance active contient un acide.

**3.** Système thérapeutique suivant les revendications 1 et 2, caractérisé en ce que l'acide est un polyacide polymérique.

**4.** Système thérapeutique suivant les revendications 1 et 2, caractérisé en ce que la couche dépourvue de substance active contient un tampon réglé du côté acide.

**5.** Système thérapeutique suivant la revendication 1, caractérisé en ce que la substance active est un acide et la couche dépourvue de substance active contient une base.

**6.** Système thérapeutique suivant les revendications 1 et 5, caractérisé en ce que la base est une polybase polymérique.

**7.** Système thérapeutique suivant les revendications 1 et 5, caractérisé en ce que la couche dépourvue de substance active contient un tampon réglé du côté basique.

**8.** Système thérapeutique suivant la revendication 1, caractérisé en ce que la substance active est un sel et la couche dépourvue de substance active contient un échangeur d'ions.

**9.** Système thérapeutique suivant la revendication 1, caractérisé en ce que la couche dépourvue de substance active contient un adjuvant qui, en raison d'interactions physiques, peut absorber une partie de la substance active.

**10.** Système thérapeutique suivant les revendications 1 et 9, caractérisé en ce que l'adjuvant est du gel de silice, un dérivé de gel de silice, l'oxyde d'aluminium, du charbon activé, du dioxyde de titane, de l'oxyde de zinc, de l'oxyde de magnésium, du lactose ou un dérivé de la cellulose.

11. Système thérapeutique suivant les revendications 1 et 9, caractérisé en ce que la substance active est un composé organique de l'azote, de préférence la nitroglycérine.

12. Système thérapeutique suivant les revendications 1 et 9, caractérisé en ce que la substance active est la nicotine.

13. Système thérapeutique suivant les revendications 1 et 9, caractérisé en ce que la substance active est un anorexigène.

14. Système thérapeutique suivant la revendication 13, caractérisé en ce que l'anorexigène est l'un des composés qui suivent : nor-pseudoéphédrine, amfépramon, méfénorex, propylhexédrine, fenfluramine ou mazindole.

15. Système thérapeutique suivant les revendications 1 et 9, caractérisé en ce que la substance active est un β-bloqueur.

16. Système thérapeutique suivant la revendication 15, caractérisé en ce que le β-bloqueur est l'un des composés qui suivent : alprénolol, oxprénolol, penbutolol, bupranolol, métoprolol, bétaxolol, aténolol, acébutolol, métipranolol, propranolol, nadolol, pindolol, mépindolol, cartéolol, carazolol, timolol ou sotalol.

17. Système thérapeutique suivant les revendications 1 et 9, caractérisé en ce que la substance active appartient au groupe des α-sympathicomimétiques.

18. Système thérapeutique suivant la revendication 17, caractérisé en ce que la substance active est l'un des composés qui suivent : norfénétrine, octopamine, oxédrine, métaraminol, midodrine ou oxilofrine.

19. Système thérapeutique suivant les revendications 1 et 9, caractérisé en ce que la substance active appartient au groupe des β-sympathicomimétiques.

20. Système thérapeutique suivant la revendication 19, caractérisé en ce que la substance active est l'un des composés qui suivent : salbutamol, terbutaline, fénotérol, clenbutérol, réprotérol, hexoprénaline, baméthane ou isoxsuprine.

**Revendications pour les Etats contractants suivants : ES, GR**

1. Procédé de confection d'un système thérapeutique pour l'administration transdermique, ou transmucosale, retardée et régulée, de substances actives, à partir d'une couche dorsale imperméable, où sont prévus un réservoir contenant la substance active, une couche sensiblement dépourvue de substance active mais perméable à la substance active et une couche protectrice amovible et la couche dépourvue de substance active n'est mise en contact avec le réservoir à substance active qu'immédiatement avant ou après l'application et se situe alors entre le réservoir et la surface d'application, caractérisé en ce que l'on ajoute à la couche dépourvue de substance active un adjuvant qui convertit au moins une partie de la substance active qui y diffuse après contact avec le réservoir à substance active en une forme non biodisponible.

2. Procédé suivant la revendication 1, caractérisé en ce que, à titre de substance active, on utilise une base et on ajoute un acide à la couche dépourvue de substance active.

3. Procédé suivant suivant les revendications 1 et 2, caractérisé en ce que, à titre d'acide, on utilise un polyacide polymérique.

4. Procédé suivant les revendications 1 et 2, caractérisé en ce qu'on utilise un tampon réglé du côté acide dans la couche dépourvue de substance active.

5. Procédé suivant la revendication 1, caractérisé en ce que, à titre de substance active, on utilise un acide et on ajoute une base à la couche dépourvue de substance active.

6. Procédé suivant les revendications 1 et 5, caractérisé en ce que, à titre de base, on utilise une polybase polymérique.

7. Procédé suivant les revendications 1 et 5, caractérisé en ce que l'on utilise un tampon réglé du côté basique dans la couche dépourvue de substance active.

8. Procédé suivant la revendication 1, caractérisé en ce que, à titre de substance active, on utilise un sel et on ajoute un échangeur d'ions à la couche dépourvue de substance active..

9. Procédé suivant la revendication 1, caractérisé en ce qu'à la couche dépourvue de substance active on ajoute un adjuvant qui, en raison d'interactions physiques, peut absorber une partie de la substance active.

10. Procédé suivant les revendications 1 et 9, caractérisé en ce que, à titre d'adjuvant, on ajoute du gel de silice, un dérivé de gel de silice, de l'oxyde d'aluminium, du charbon activé, du dioxyde de titane, de l'oxyde de zinc, de l'oxyde de magnésium, du lactose ou un dérivé de la cellulose.

11. Procédé suivant les revendications 1 et 9, caractérisé en ce que, à titre de substance active, on utilise un composé organique de l'azote, de préférence la nitroglycérine.

12. Procédé suivant les revendications 1 et 9, caractérisé en ce que, à titre de substance active, on utilise la nicotine.

13. Procédé suivant les revendications 1 et 9, caractérisé en ce que, à titre de substance active, on utilise un anorexigène.

14. Procédé suivant la revendication 13, caractérisé en ce que, à titre d'anorexigène, on utilise l'un des composés qui suivent : nor-pseudoéphédrine, amfépramon, méfénorex, propylhexédrine, fenfluramine ou mazindole.

15. Procédé suivant les revendications 1 et 9, caractérisé en ce que, à titre de substance active, on utilise un bêta-bloqueur.

16. Procédé suivant la revendication 15, caractérisé en ce que, à titre de bêta-bloqueur, on utilise l'un des composés qui suivent : alprénolol, oxprénolol, penbutolol, bupranolol, métoprolol, bétaxolol, aténolol, acébutolol, métipranolol, propranolol, nadolol, pindolol, mépindolol, cartéolol, carazolol, timolol ou sotalol.

17. Procédé suivant les revendications 1 et 9, caractérisé en ce qu'on utilise une substance active qui appartient au groupe des alpha-sympathicomimétiques.

18. Procédé suivant la revendication 17, caractérisé en ce que, à titre de substance active, on utilise l'un des composés qui suivent : norfénétrine, octopamine, oxédrine, métaraminol, midodrine ou oxilofrine.

19. Procédé suivant les revendications 1 et 9, caractérisé en ce qu'on utilise une substance active qui appartient au groupe des bêta-sympathicomimétiques.

20. Procédé suivant la revendication 19, caractérisé en ce que, à titre de substance active, on utilise l'un des composés qui suivent : salbutamol, terbutaline, fénotérol, clenbutérol, réprotérol, hexoprénaline, baméthane ou isoxsuprine.

FIG. 1

EP 0 384 267 B1

[mg/ 16 cm]]

Wirkstoff: Nitroglycerin

FIG. 2